# EUROPÄISCHE PATENTSCHRIFT

(11) **EP 1 458 744 B1**
(45) Veröffentlichungstag und Bekanntmachung des Hinweises auf die Patenterteilung: **01.08.2007**
(21) Anmeldenummer: 02792892.8
(22) Anmeldetag: 04.12.2002
(51) Int. Cl.: C07K 1/113

(54) **VERFAHREN ZUR RENATURIERUNG VON PROTEINEN**
METHOD FOR RENATURATING PROTEINS
PROCEDE DE RENATURATION DE PROTEINES

(30) Priorität: 14.12.2001 DE 10161577
(43) Veröffentlichungstag der Anmeldung: 22.09.2004
(73) Patentinhaber: Scil proteins GmbH, 06120 Halle/Saale (DE)
(72) Erfinder: RUDOLPH, Rainer, 06120 Halle (DE); LILIE, Hauke, 06112 Halle (DE); RAUE, Uta, 06217 Merseburg (DE)
(74) Vertreter: Behnisch, Werner
(86) Internationale Anmeldenummer: PCT/EP2002/013732
(87) Internationale Veröffentlichungsnummer: WO 2003/051908

(56) Entgegenhaltungen:
- US-A- 4 961 969
- RUDOLPH R ET AL: "IN VITRO FOLDING OF INCLUSION BODY PROTEINS" FASEB JOURNAL, FED. OF AMERICAN SOC. FOR EXPERIMENTAL BIOLOGY, BETHESDA, MD, US, Bd. 10, Nr. 1, 1996, Seiten 49-56, XP000605154
- YEH S R ET AL: "Hierarchical folding of cytochrome c." NATURE STRUCTURAL BIOLOGY. UNITED STATES JUN 2000, Bd. 7, Nr. 6, Juni 2000 (2000-06), Seiten 443-445, XP009009665 in der Anmeldung erwähnt
- REHM BERND H A ET AL: "Matrix-assisted in vitro refolding of Pseudomonas aeruginosa class II polyhydroxyalkanoate synthase from inclusion bodies produced in recombinant Escherichia coli." BIOCHEMICAL JOURNAL, Bd. 358, Nr. 1, 2001, Seiten 263-268, XP002238953

## Beschreibung

Die Erfindung betrifft die Verwendung von substituierten Imidazoliumsalzen zur Renaturierung, zur Verminderung der Aggregation und/oder zur Erhöhung der thermischen Stabilität von Proteinen sowie ein Verfahren zur Renaturierung, zur Verminderung der Aggregation und/oder zur Erhöhung der thermischen Stabilität von Proteinen sowie renaturierte Proteine, die mit dem erfindungsgemäßen Verfahren hergestellt wurden.

Unter Renaturierung von Proteinen wird in diesem Zusammenhang die Gewinnung oder Wiederherstellung der biologischen Aktivität und/oder der weitgehend korrekten Faltung von Proteinen angesehen. Dem Problem der Proteinrenaturieirung kommt in der Biotechnologie eine große Bedeutung zu. Aufgrund der Entwicklungen in der Molekularbiologie ist es möglich, Proteine, ausgehend von deren codierenden Sequenzen zu klonieren und rekombinant in Wirtsorganismen zu produzieren. Hierbei fallen die rekombinanten Proteinen allerdings häufig als biologisch inaktive Aggregate, den inclusion bodies, an. Das in den inclusion bodies enthaltene Protein muß anschließend durch Renaturierung in vitro in die biologisch aktive Form überführt werden.
Diese Renaturierung ist ein sehr komplexer strukturbiologischer Prozeß, der häufig nur in sehr geringen Ausbeuten an nativem Protein mündet. Bereits Steigerungen der Ausbeute an renaturiertem Protein von wenigen Prozent stellen daher eine wesentliche Verbesserung und einen wirtschaftlichen Vorteil dar.

Bei der *in vitro* Faltung von *inclusion body* Proteinen beobachtet man meist eine kinetische Konkurrenz zwischen der korrekten Faltung und unproduktiven Nebenreaktionen wie Fehlfaltung und Aggregation. Die Aggregationsprozesse beruhen meist auf hydrophoben Wechselwirkungen der weitgehend entfalteten Polypeptidketten oder nichtnativer, partiell gefalteter Intermediate. Unabhängig von ihrer molekularen Ursache weisen diese Aggregationsprozesse eine Reaktionsordnung von > 2 auf. Dementsprechend erhöht sich die Geschwindigkeit der unproduktiven Aggregationsreaktionen bei Erhöhung der Konzentration der entfalteten Polypeptidkette. Da die korrekten Faltungsprozesse meist durch Reaktionen erster Ordnung bestimmt werden, bedeutet dies, dass die Aggregationsreaktionen bei Erhöhung der Konzentration an denaturiertem Protein über die korrekte Faltung dominieren. Aus diesem Grund werden *in vitro* Faltungsreaktionen meist bei sehr niedriger Proteinkonzentration durchgeführt. Zusätzlich müssen physikalische Parameter wie pH, Ionenstärke, Redoxsystem und Temperatur für die Renaturierung von Proteinen optimiert werden. Allerdings führt auch dies häufig nicht zu dem gewünschten Erfolg.

Weiterhin ist schon seit langem bekannt, dass Pufferzusätze wie Harnstoff oder Guanidin in nicht denaturiender Konzentration einen positiven Einfluß auf die Faltungseffizienz haben können. Als Alternative zu Guanidin oder Harnstoff wurden auch andere chaotrope Stoffe wie Alkylharnstoff oder organische Cosolvenzien wie beispielsweise Carbonsäureamide oder alkylierte Amine bei *in vitro* Faltungsprozessen verwendet.

Im Falle der Renaturierung von humanem Gewebsplasminogenaktivator aus *E*. *coli inclusion bodies* wurde beobachtet, dass die Ausbeute bei der *in vitro* Faltung durch Zusatz von L-Arginin gesteigert werden kann. Weitere Studien haben gezeigt, dass auch bei der *in vitro* Faltung anderer Proteine wie Antikörperfragmente oder Immunotoxine die Faltungseffizienz durch L-Arginin verbessert werden kann. Obgleich Arginin eine Guanidinogruppe enthält, wirkt es nicht so stark strukturdestabilisierend wie Guanidin. Dementsprechend erhöht Arginin vermutlich die Löslichkeit von entfalteten Polypeptidketten oder von Faltungsintermediaten, ohne dass es die native Proteinstruktur signifikant destabilisiert. Weitere Additive zur Erhöhung der *in vitro* Faltungseffizienz sind hochkonzentrierte Tris-Puffer, Polyethylenglycol oder Detergenzien. Durch Verwendung gemischter Mizellen (beispielsweise aus Triton X 100 und Phospholipiden) wurden ebenfalls verbesserte Faltungsausbeuten erzielt. Diese gemischten Mizellen enthalten sowohl polare als auch unpolare Gruppen, die vermutlich durch Wechselwirkungen mit Faltungsintermediaten deren Aggregation unterdrücken.
In der Veröffentlichung Amersham Pharmacia Biotech; Data File: Affinity Chromatography; HiTrap Chelating 1 ml und 5 ml; 18-1134-78 AB, S. 1-6; 05.2000 ist eine Renaturierung eines His₆-getaggten Proteins auf einer Ni²⁺-NTA-Säule beschrieben. Im Renaturierungspuffer sind 20 mM Imidazol enthalten. Diese Konzentration an Imidazol unterbindet unspezifische Wechselwirkungen zwischen Proteinen und der Säulenmatrix, bewirkt aber keine Renaturierung. Imidazol ist vielmehr als Denaturierungsmittel bekannt.

In Yeh et al., Hierachical folding of cytochrome c; Nature Struct. Biol. 7, S. 443-445, 2000, ist beschrieben, daß Imidazol die Kinetik der Faltung des Proteins Cytochrom C beschleunigen kann. Durch Imidazol wurde die intramolekulare Wechselwirkung von His33 des Proteins mit seinem kovalent gebundenen Liganden unterdrückt. Imidazol in höheren Konzentrationen wirkt grundsätzlich denaturierend auf Proteine.

Trotz einer Vielzahl von in der Literatur angegebenen Protokollen zur Renaturierung von Proteinen ist die industrielle Nutzung dieser Technik noch nicht weit vorangeschritten. Die Gründe hierfür liegen sowohl in den technischen als auch biochemischen Schwierigkeiten. Da auch unter optimalen Bedingungen die Renaturierung von Proteinen nur bei sehr niedrigen Proteinkonzentrationen möglich ist, erfordert eine industrielle Produktion die Aufarbeitung sehr großer Renaturierungsvolumen. Darüber hinaus ist auch unter optimierten Bedingungen die Renaturierungseffizienz vieler Proteine nur gering.

Aufgabe der Erfindung ist daher die Identifizierung von Substanzen, die die Effizienz der Proteinfaltung in vitro steigern und ein Verfahren bereitzustellen, mit dem Proteine effektiv und in größerer Ausbeute als bisher renaturiert werden können.

Gelöst wird diese Aufgabe erfindungsgemäß durch die Verwendung von substituierten Imidazoliumsalzen zur Renaturierung, zur Verminderung der Aggregation und/oder zur Erhöhung der thermischen Stabilität von Proteinen sowie durch ein Verfahren zur Renaturierung, zur Verminderung der Aggregation und/oder zur Erhöhung der thermischen Stabilität von Proteinen, bei dem die zu behandelnden Proteine mit einem flüssigen Medium, welches substituierte Imidazoliumsalze enthält, in Kontakt gebracht werden. Die vorliegende Erfindung betrifft auch Proteine, die mit dem erfindungsgemäßen Verfahren hergestellt wurden.

Die bevorzugte erfindungsgemäße Verwendung ist die Verwendung zur Renaturierung von Proteinen.

Unter Verminderung der Aggregation ist erfindungsgemäß zu verstehen, dass die Aggregation von Proteinen, die üblicherweise bei längeren Aufbewahrungszeiträumen in flüssigen Medien auftritt und womit ein Verlust oder eine Verminderung der biologischen Funktionsfähigkeit einhergeht, vermindert oder sogar weitgehend verhindert wird.

Unter Erhöhung der thermischen Stabilität ist erfindungsgemäß zu verstehen, dass die biologische Aktivität bzw. die korrekte Proteinfaltung auch bei Temperaturen bis weit oberhalb Raumtemperatur über längere Zeit beibehalten werden kann.

Bei den erfindungsgemäß verwendeten Imidazoliumsalzen handelt es sich bevorzugt um bei Raumtemperatur ionische Flüssigkeiten. Falls es sich nicht um bei Raumtemperatur flüssige Verbindungen handelt, sollten die erfindungsgemäßen Imidazoliumsalze zumindest bei den Behandlungsbedingungen in flüssiger Form vorliegen und/oder in dem flüssigen Medium löslich sein. Bei den im Rahmen der vorliegenden Erfindung verwendeten Imidazoliumsalzen ist die positive Ladung der organischen Komponente mit dem Ringsystem assoziiert und üblicherweise und bevorzugt in dem Imidazoliumring delokalisiert.

Das Verfahren beruht auf der überraschenden Entdeckung, dass substituierte Imidazoliumsalze die Aggregation von Proteinen unterdrücken und die Effizienz der Renaturierung steigern. Bisher wurden diese organischen Salze lediglich als Lösungsmittel in der organischen Synthese und der Zwei-Phasen-Katalyse eingesetzt, ein Einfluss auf strukturbildende Prozesse in der Biochemie war bislang unbekannt. Auch für andere organische Salze ist beschrieben worden, dass sie eine effizientere Renaturierung von Proteinen ermöglichen, wie z.B. 3-(1-Pyridinio)-1-propansulfat oder Trigonellin-Hydrochlorid (WO 99/18196). Diese Substanzen weisen bspw. keine delokalisierte positive Ladung im heterocyclischen Ringsystem auf. Im direkten Vergleich ihrer Wirkung auf die Renaturierung von Proteinen weisen die Imidazoliumderivate der vorliegenden Erfindung eine deutlich höhere Effizienz auf (vgl. auch die Ausführungsbeispiele sowie Abb. 2 und 3 mit Abb. 8 und 9).

Es ist bevorzugt, dass die Imidazoliumringe der Imidazoliumsalze der vorliegenden Erfindung durch Alkyl-, Alkenyl- Aryl- und/oder Aralkyl-Reste substituiert sind, welche wiederum durch funktionelle Gruppen substituiert sein können, bspw. durch Stickstoff-, Schwefel-, und/oder Phosphor-haltige Gruppen, wobei verschiedene Oxidationsstufen möglich sind Erfindungsgemäß bevorzugte Beispiele für diese funktionellen Gruppen sind: Amin-, Carboxyl-, Carbonyl, Aldehyd-, Hydroxy-, Sulfat-, Sulfonat- und/oder Phosphatgruppen.

Erfindungsgemäß können ein oder beide N-Atome des Imidazoliumrings durch gleiche oder unterschiedliche Substituenten substituiert sein. Vorzugsweise sind beide Stickstoffatome des Imidazoliumrings durch gleiche oder unterschiedliche Substituenten substituiert.

Es ist erfindungsgemäß auch möglich und bevorzugt, dass die Imidazoliumsalze zusätzlich oder ausschließlich an einem oder weiteren der Kohlenstoffatome des Imidazoliumrings substituiert sind.

Als Substituenten kommen bevorzugt C₁ - C₄-Alkylreste in Frage, z.B. Methyl-, Ethyl-, n-Propyl-, Isopropyl-, n-Butyl und/oder Isobutylreste, besonders bevorzugt Ethyl- und/oder Methylreste. Ebenfalls bevorzugte Substituenten sind C₂-C₄- Alkenylreste, wie Ethylen-, n-Propylen, Isopropylen, n-Butylen und/oder Isobutylen. Bei diesen C₁-C₄-Alkylresten und C₂-C₄ Alkenylresten weisen die erfindungsgemäßen lmidazolium-verbindungen eine erfindungsgemäß besonders geeignete Wasserlöslichkeit auf, die bei längeren Alkyl- oder Alkenylketten abnimmt. Die Wasserlöslichkeit kann aber durch löslichkeitsfördernde Substituenten an den Alkyl- oder Alkenylketten wiederum verbessert werden, bspw. durch Sulfat- Sulfonat- , Amino- oder Phosphatreste. Erfindungsgemäß sind auch Alkyl und Alkenylsubstituenten mit mehr als 4 C-Atomen umfasst, wobei bspw. C₅-C₁₀-Alkyl- oder Alkenylsubstituenten ebenfalls noch bevorzugt sind. Diese C₅-C₁₀-Alkyl- oder Alkenylreste sind weiterhin bevorzugt, wenn sie ein oder mehrere weitere Substituenten an den Alkyl- und/oder Alkenylgruppen aufweisen, wie Phosphat-, Sulfonat-, Amino- und/oder Phosphatgruppen.

Als Arylsubstituenten sind erfindungsgemäß mono- und/oder bicyclische Arylreste bevorzugt, Phenyl, Biphenyl und/oder Naphthalin sowie Derivate dieser Verbindungen mit Hydroxy-, Sulfonat-, Sulfat-, Amino, Aldehyd-, Carbonyl- und/oder Carboxygruppen. Beispiele für bevorzugte Arylsubstituenten sind Phenol, Biphenyl, Biphenol, Naphthalin, Naphthalincarbonsäuren, Naphthalinsulfonsäuren, Biphenylole, Biphenylcarbonsäuren, Phenol, Phenylsulfonat, und/oder Phenolsulfonsäuren.

Bevorzugte Aralkylreste sind Benzyl- oder substituierte Benzylreste.

Es ist besonders bevorzugt, dass der Imidazoliumring an mindestens einem N-Atom einen Methylrest aufweist.

Die erfindungsgemäß verwendeten Imidazoliumsalze sind bevorzugt Flüssigkeiten, d.h. es handelt sich bevorzugt um bei Raumtemperatur (ca. 25°C) ionische Flüssigkeiten. Es können jedoch ebenso bei Raumtemperatur nicht flüssige Imidazoliumsalze verwendet werden, die aber bei den Renaturierungsbedingungen in flüssiger Form vorliegen sollten bzw. in dem flüssigen Medium löslich sein sollten.

Imidazoliumsalze unterscheiden sich in ihren Eigenschaften deutlich von Imidazol. Insbesondere sind Imidazoliumsalze hydrophil, wo hingegen Imidazol hydrophob ist und dieses auf Proteine denaturierend wirkt.

Als anionische Gegenionen für die substituierten Imidazoliumringe sind erfindungsgemäß insbesondere Halogene und halogenhaltige Ionen geeigent. Besonders bevorzugt sind dabei Chlorid und Tetrafluorborat. Weiterhin bevorzugt sind Phosphat, Sulfat und Isocyanat.

Erfindungsgemäß besonders bevorzugt verwendbare Imidazoliumsalze sind: 1-Ethyl-3-methyl-imidazolium-tetrafluorborat, 1-Ethyl-3-methyl-imidazolium-chlorid, 1-Butyl-3-methyl-imidazolium-tetrafluorborat.

Erfindungsgemäß können bevorzugt die nachfolgenden Proteinklassen und/oder Proteine renaturiert werden, deren thermische Stabilität erhöht werden und/oder deren Aggregation vermindert werden bzw. die nachfolgenden Proteine und/oder Proteinklassen können in dem erfindungsgemäßen Verfahren bevorzugt eingesetzt werden:
Proteasen, bevorzugt Serin-Proteasen, insbesondere Thrombin, Faktor Xa, Caspasen, Cathepsine, Trypsin und Chymotrypsin, Cystein-Proteasen, saure Proteasen, wie Pepsin oder Rennin, Metallo-Proteasen, wie Thermolysin; Proteaseinhibitoren, bevorzugt Pepstatine, Antipain, Chemostatine, Elastinal, Leupeptine, Bestatin, Antithrombin III;
DNA-bindende Proteine, bevorzugt Transkriptionsfaktoren, insbesondere NF Kappa B und Mitglieder der Familien Jun, Fos, Krox, Myc, E2F, virale T-Antigene;
virale Proteine, z.B. virale Hüllproteine, Kapsidproteine, virale Proteasen, Polymerasen und/oder T-Antigene;
Phosphatasen;
Proteinkinasen, bevorzugt Tyrosinkinasen und/oder Serinkinasen;
Proteine der Immunglobulin-Superfamilie, z.B. Antikörper und Fragmente hiervon; Wachstumsfaktoren, z.B. epidermaler Wachstumsfaktor (EGF), Erythropoeietin, Fibroblasten-Wachstumsfaktor (FGF), Insulin-ähnliche Wachstumsfaktoren I und II (IGF I und IGF II), Interlaukin-2 (IL-2), Nervenwachstumsfaktor (NGF), transformierender Wachstumsfaktor-β (TGF- β) und/oder Thrombocyten-Wachstumsfaktor (PDGF)
sowie von diesen Proteinen abgeleitete Proteine und Fragmente.

Erfindungsgemäß können disulfidfreie und disulfidverbrückte Proteine renaturiert werden und/oder deren thermische Stabilität verbessert und/oder deren Aggregation verhindert bzw. vermindert werden. Besonders bevorzugt können Mehrdomänenproteine und komplex disulfidverbrückte Proteine behandelt werden wie z.B. Lysozym, rPA (rekombinanter Plasminogenaktivator, Handelsname Replase), alpha-Glucosidase, Antikörper und von ihnen abgeleitete Fragmente und/oder Wachstumsfaktoren. Diese Proteine/Proteinklassen dienen lediglich als Beispiele, die Erfindung ist jedoch nicht auf diese Liste beschränkt.

Bei dem erfindungsgemäßen Verfahren zur Renaturierung, zur Verminderung der Aggregation und/oder zur Erhöhung der thermischen Stabilität von Proteinen wird als flüssiges Medium bevorzugt ein für das zu behandelnde Protein geeignetes Puffersystem eingesetzt. Als Puffersubstanzen sind Tris, Hepes, Mes, Mops, Acetat, Glycin und/oder Phosphat bevorzugt einsetzbar, wobei die Pufferkonzentration in dem flüssigen Medium bevorzugt 10- 1000 mM, bevorzugter 5-200 mM, ebenfalls bevorzugt 10 - 200 mM und weiterhin bevorzugt 10-50 mM beträgt. Der pH-Wert des Puffersystems beträgt vorzugsweise 4-11, weiterhin bevorzugt 7,5 - 10,5. Für Lysozym beträgt der pH-Wert bei der Renaturierung vorzugsweise etwa 8, und für die Renaturierung von rPA beträgt der pH-Wert vorzugsweise etwa 10,5. Für andere zu renaturierende Proteine können die Pufferzusammensetzungsparameter individuell angepaßt und optimiert werden, so dass eine maximale Ausbeute an renaturiertem Protein erhalten wird.

Bei dem erfindungsgemäßen Verfahren Renaturierung von Proteinen erfolgt das Inkontaktbringen der zu reanurierenden bzw. zu behandelnden Proteine mit dem flüssigen Medium bevorzugt, indem das zu behandelnde Protein mit dem flüssigen Medium verdünnt, dialysiert und/oder diafiltriert wird. Grundsätzlich sollte bei einer Renaturierung eine Umpufferung des denaturierten Proteins in den Renaturierungspuffer gewährleistet sein.

Beim Inkontaktbringen, insbesondere bei der Renaturierung, beträgt die Proteinkonzentration des zu behandelnden Proteins bevorzugt 5 - 500 µg/ml, bevorzugt 10 - 100 µg/ml, noch bevorzugter ungefähr 50 - 100 µg/ml. Diese Werte können von einem Fachmann für das jeweilige zu renaturierende bzw. zu behandelnde Protein an die Löslichkeitseigenschaften des Proteins angepasst werden.

Das erfindungsgemäße Verfahren eignet sich zur Renaturierung von disulfidfreien und disulfidverbrückten Proteinen. Dabei werden disulfidfreie Proteine bevorzugt in Gegenwart eines Reduktionsmittels, wie DDT, DTE und/oder Cystein, bevorzugt in einer Konzentration von 1 - 10 mM, mit dem Renaturierungsmedium, welches substituierte Imidazoliumsalze enthält, in Kontakt gebracht.

Bei der Renaturierung disulfidverbrückter Proteine werden diese bevorzugt in Gegenwart eines Redoxsystems aus reduzierten und oxidierten Thiolsubstanzen wie DTT, DTE, Glutathion, Cystein, Mercaptoethanol, bevorzugt in einer Konzentration von 1-10 mM, in Kontakt gebracht. Die Konzentrationsverhältnisse von reduzierten Substanzen zu oxidierten Substanzen ("reduziert : oxidiert") betragen dabei vorzugsweise 1:10 bis 20: 1, bevorzugt 1:5-10 :1, weiterhin bevorzugt 1:1 bis 5:1.

Die Konzentration an substituierten Imidazoliumsalzen beträgt beim Inkontaktbringen, insbesondere bei der Renaturierung, bezogen auf das Renaturierungsmedium, bevorzugt 5-95 Vol.-%, weiterhin bevorzugt 5-50 Vol.-%, weiterhin bevorzugt 10-40 Vol.-%. Auch hier kann die optimale Konzentration für das jeweilige Protein durch einfache Versuche ermittelt werden. Üblicherweise ergibt der Zusatz von substituierten Imidazoliumsalzen in einem Anteil von 10-40 Vol.-% die besten Renaturierungsausbeuten. Wie bereits erwähnt, kann jedoch, abhängig von den jeweils eingesetzten Imidazoliumverbindungen, die Konzentration bis zu 95 Vol.-% betragen.

In Molaritäten ausgedrückt entsprechen die oben genannten Vol.-% Angaben von 10-40 Vol.-% etwa 0,5 - 3 M: Für den Fall von 1-Ethyl-3-Methyl-Imidazoliumchlorid genau 0,68-2,73 M. In Molaritäten ausgedrückt, ist daher erfindungsgemäß bevorzugt, daß die Molaritäten substituierter Imidazoliumsalze, bezogen auf das Renaturierungsmedium, etwa 0,25 - 5 M betragen, weiterhin bevorzugt 0,25 - 3,5 M und am bevorzugtesten etwa 0,5 - 3 M.

Die Renaturierungsdauer beträgt üblicherweise bevorzugt: 0,1 -100 h, weiterhin bevorzugt 1 -50 h, noch bevorzugter ungefähr 2-20 h.

Die Renaturierung erfolgt vorzugsweise bei niedrigen Temperaturen von 0 - 37°C, bevorzugt 5 - 15°C, da bei höheren Temperaturen die Aggregationsreaktionen zunehmen.

Als Parameter der Optimierung der Renaturierung können bevorzugt die biologische Aktivität des Proteins und das Aggregationsverhalten über den Prozeßverlauf gemessen werden.

In einer weiteren Ausführungsform des erfindungsgemäßen Verfahrens kann das Protein pulsweise mehrfach hintereinander oder kontinuierlich dem Renaturierungspuffer zugegeben werden.

Insgesamt ist das erfindungsgemäße Verfahren besonders vorteilhaft zur Renaturierung von Proteinen geeignet. Dabei ist es weiterhin bevorzugt, dass das flüssige Medium für die Renaturierung von Proteinen neben den substituierten Imidazoliumsalzen weitere bekannte Renaturierungssubstanzen oder die Renaturierung unterstützende Substanzen enthält, wie z.B. Harnstoff, Guanidin, L-Arginin, Alkylharnstoff, Carbonsäureamide, alkylierte Amine, Tris-Puffer, Polyethylenglykol und/oder Detergenzien.

Die vorliegende Erfindung wird im folgenden anhand von Ausführungsbeispielen beschrieben. Dabei wird auf die folgenden Figuren Bezug genommen:

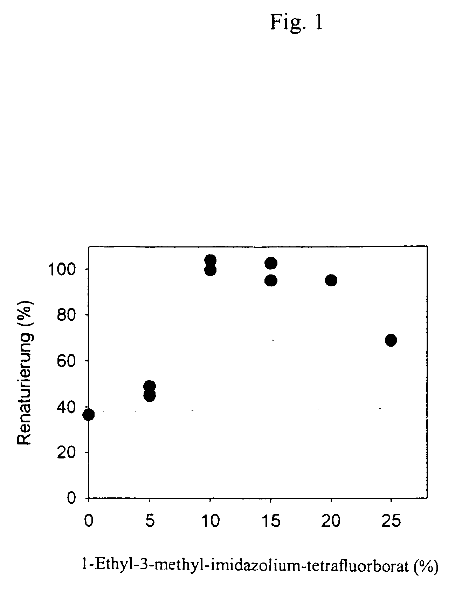

- Figur 1:: zeigt die Renaturierungsausbeute in % für Lysozym in Abhängigkeit von der Konzentration an 1-Ethyl-3-methyl-imidazolium-tetrafluorborat;

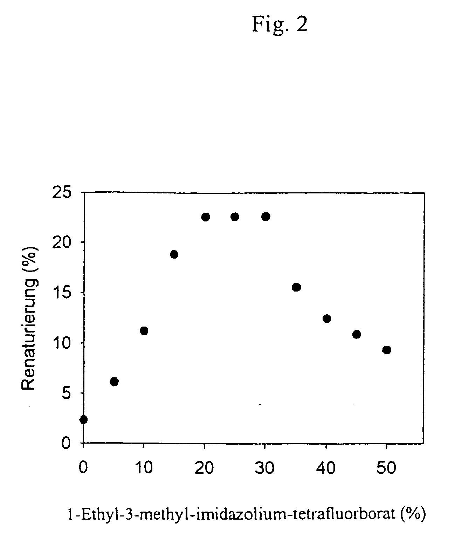

- Figur 2:: zeigt die Renaturierungsausbeute von rPA in Abhängigkeit von der Konzentration an 1-Ethyl-3-methyl-imidazolium-tetrafluorborat;

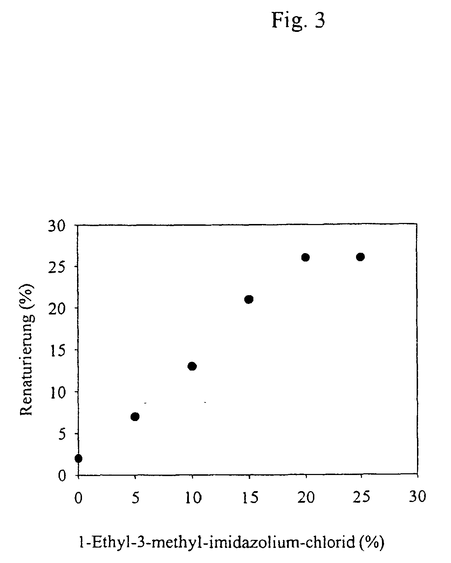

- Figur 3:: zeigt die Renaturierungsausbeute von rPA in Abhängigkeit von der Konzentration an 1-Ethyl-3-methyl-imidazolium-chlorid;

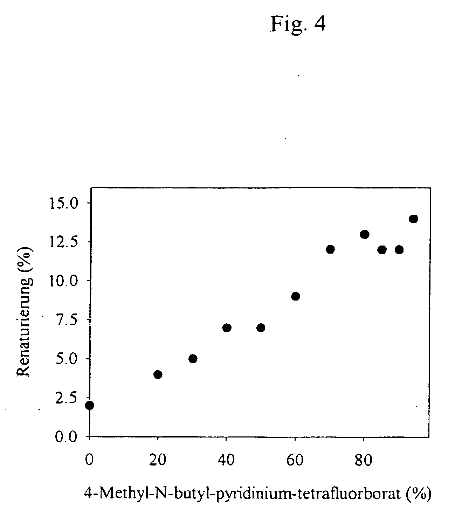

- Figur 4: zeigt die Renaturierungsausbeute von rPA in Abhängigkeit von der Konzentration an 4-Methyl-N-butyl-pyridinium-tetrafluorborat;

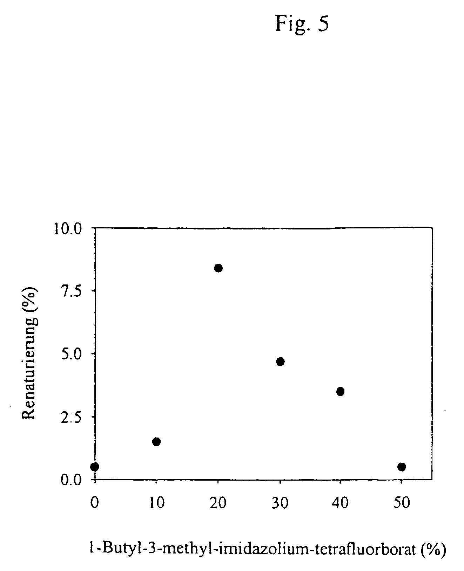

- Figur 5:: zeigt die Renaturierungsausbeute von rPA in Abhängigkeit von der Konzentration an 1-Butyl-3-methyl-imidazolium-tetrafluorborat;

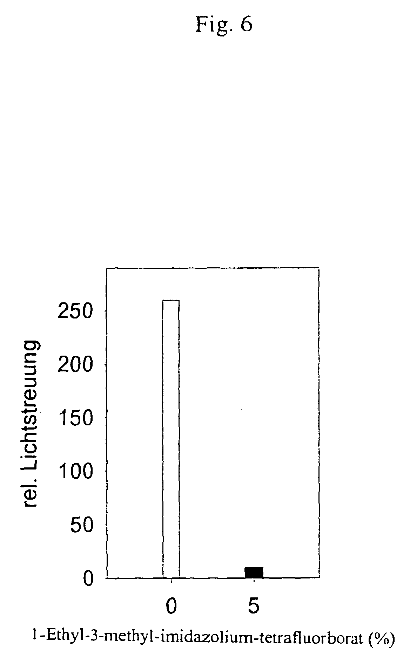

- Figur 6:: ist eine graphische Darstellung der Aggregation von rPA während der Renaturierung, bestimmt mittels Lichtstreuung, wobei 0% und 5% 1-Ethyl-3-methyl-imidazolium-tetrafluorborat, enthalten sind.

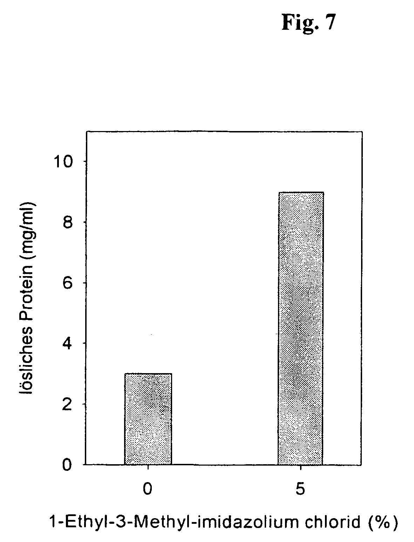

- Figur 7:: zeigt die Konzentration an gelöstem nGLP1R bei Abwesenheit und in Gegenwart von 5 % 1-Ethyl-3-methyl-imidazoliumchlorid.

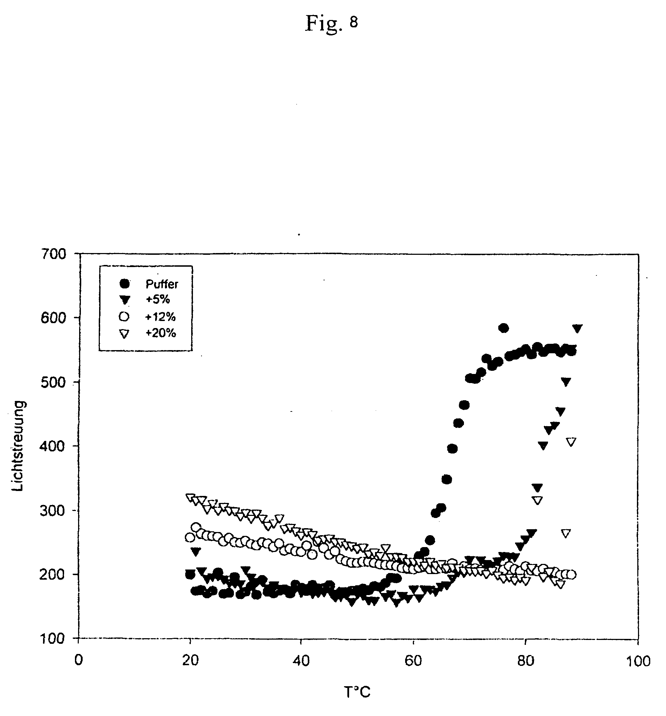

- Figur 8:: ist eine graphische Darstellung des Aggregationsverhaltens von rPA in Abhängigkeit der Temperatur und für verschiedene Konzentrationen an 1-Ethyl-3-methyl-imidazolium-tetrafluorborat, bestimmt mittels Lichtstreuung;

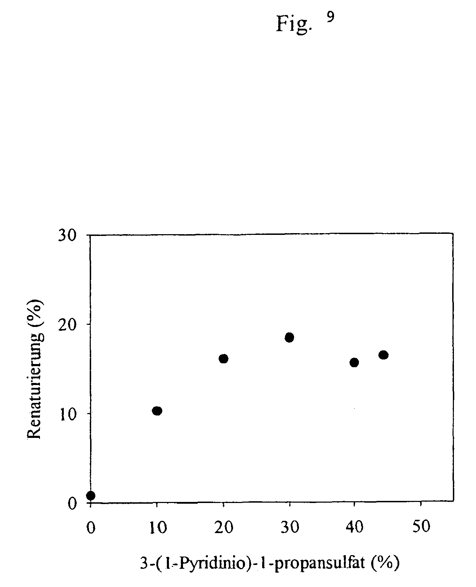

- Figur 9:: zeigt die Renaturierungsausbeute von rPA in Abhängigkeit von der Konzentration an 3-(1-Pyridinio)-1-propansulfat ;

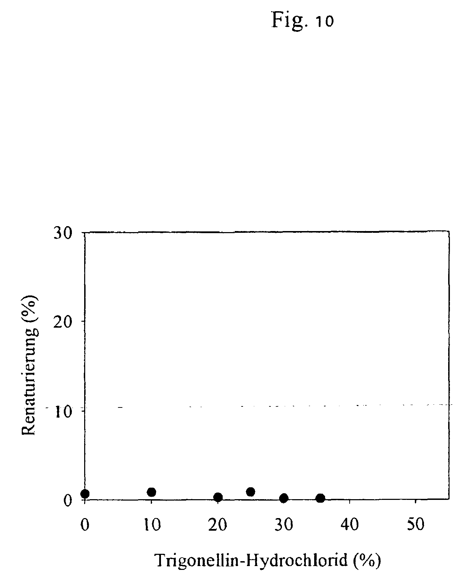

- Figur 10:: zeigt die Renaturierungsausbeute von rPA in Abhängigkeit von der Konzentration an Trigonellin-hydrochlorid ;

### Beispiel 1: Denaturierung von Proteinen

Lysozym, rPA oder inclusion body Proteine werden mit einer Proteinkonzentration von 0,5-20 mg/ml in 0,1 M Tris, pH 8, 6 M Guanidinhydrochlorid, 1 mM EDTA, 200 mM DTT für mindestens 2 h bei Raumtemperatur denaturiert und reduziert. Anschließend wird der pH-Wert durch Zugabe von HCI auf ca. pH 2 abgesenkt. Wahlweise kann das denaturierte Protein gegen das 1000fache Volumen 6 M Guanidinhydrochlorid, pH 2 dialysiert werden. Die Konzentration des denaturierten Proteins wird mit Hilfe des Bradford-Assay (Bradford, 1976) oder spektroskopisch bestimmt. Hierfür wird für rPA ein Extinktions-koeffizient von e(1mg/ml, 280 nm, 1 cm) = 1 und für Lysozym e(1mg/ml, 280 nm, 1cm) = 2,37 verwendet.

### Beispiel 2: Renaturierung von Lysozym

Die Renaturierung von Lysozym erfolgt durch eine 1:100 Verdünnung des denaturiert, reduzierten Proteins in den auf 10°C vortemperierten Renaturierungspuffer, die Endkonzentration des Proteins beträgt 200 µg/ml. Als Renaturierungspuffer wird 0.05 M Tris, pH 8, 1 mM ED-TA, 4 mM GSSG (oxidiertes Glutathion) und unterschiedliche Konzentrationen der ionischen Flüssigkeit 1-Ethyl-3-methyl-imidazolium-tetrafluorborat eingesetzt. Als Reduktionsmittel dient 2 mM DTT, welches durch die Verdünnung aus dem Denaturierungsansatz eingeschleppt wird. Nach der Renaturierung werden die Proben gegen das 1000fache Volumen von 0,05 M Tris, pH 8, 1 mM EDTA über Nacht dialysiert. Als Maß der Renaturierung wird die Enzymaktivität von Lysozym photometrisch gemessen und anhand einer Eichgerade quantifiziert.

In Figur 1 ist die Abhängigkeit der Renaturierungsausbeute von Lysozym von der Konzentration des Salzes 1-Ethyl-3-methyl-imidazolium-tetrafluorborat dargestellt. In Gegenwart von 10-15 Vol.-% 1-Ethyl-3-methyl-imidazollum-tetrafluorborat kann Lysozym quantitativ, also zu 100%, oxidativ renaturiert werden.

### Beispiel 3: Renaturierung von rPA mittels 1-Ethyl-3-methyl-imidazolium-tetrafluorborat

Die Renaturierung des in 6 M Guanidin, pH 2 befindlichen rPA erfolgt durch eine 1:100 Verdünnung in 0,1 M Tris, pH 10,5, 1 mM EDTA, 5 mM GSSG, 2 mM GSH (reduziertes Glutathion) in Gegenwart unterschiedlicher Konzentrationen von 1-Ethyl-3-methyl-imidazolium-tetrafluorborat. Die Renaturierung wird für mindestens 16 h bei 10°C durchgeführt. Nach der Renaturierung werden die Proben gegen das 1000fache Volumen von 0,1 M Tris, pH 8, 1 mM EDTA über Nacht dialysiert. Als Maß der Renaturierung wird die Enzymaktivität von rPA mit Hilfe des Chromozym-tPA-Assays (Roche Diagnostics) ermittelt und anhand einer Eichgerade für das native rPA quantifiziert.

In Figur 2 ist die Renaturierungsausbeute von rPA in Abhängigkeit von der Konzentration des Salzes 1-Ethyl-3-methyl-imidazolium-tetrafluorborat dargestellt. Die Renaturierungsausbeute liegt bei 23 % in Gegenwart von 20 - 30 Vol.-% 1-Ethyl-3-methyl-imidazolium-tetrafluorborat.

### Beispiel 4: Renaturierung von rPA mittels 1-Ethyl-3-methyl-imidazolium-chlorid

Die Renaturierung des in 6 M Guanidin, pH 2 befindlichen rPA erfolgt durch eine 1:100 Verdünnung in 0,1 M Tris, pH 10,5, 1 mM EDTA, 5 mM GSSG, 2 mM GSH in Gegenwart unterschiedlicher Konzentrationen von 1-Ethyl-3-methyl-imidazolium-chlorid. Die Renaturierung wird für mindestens 16 h bei 10°C durchgeführt. Nach der Renaturierung werden die Proben gegen das 1000fache Volumen von 0,1 M Tris, pH 8, 1 mM EDTA über Nacht dialysiert. Als Maß der Renaturierung wird die Enzymaktivität von rPA mit Hilfe des Chromozym-tPA-Assays (Roche Diagnostics) ermittelt und anhand einer Eichgerade für das native rPA quantifiziert.

In Figur 3 ist die Renaturierungsausbeute von rPA in Abhängigkeit von der Konzentration des Salzes 1-Ethyl-3-methyl-imidazolium-chlorid dargestellt. Die Renaturierungsausbeute liegt bei 26 % in Gegenwart von 20 - 25 Vol.-% 1-Ethyl-3-methyl-imidazolium-chlorid.

### Beispiel 5: Renaturierung von rPA mittels 4-Methyl-N-butyl-pyridinium-tetrafluorborat (Vergleichsbeispiel)

Die Renaturierung des in 6 M Guanidin, pH 2 befindlichen rPA erfolgt durch eine 1:100 Verdünnung in 0,01 M Tris, pH 10,5, 1 mM EDTA, 5 mM GSSG, 2 mM GSH in Gegenwart unterschiedlicher Konzentrationen von 4-Methyl-N-butyl-pyridinium-tetrafluorborat. Die Renaturierung wird für mindestens 16 h bei 10°C durchgeführt. Nach der Renaturierung werden die Proben gegen das 1000fache Volumen von 0,1 M Tris, pH 8, 1 mM EDTA über Nacht dialysiert. Als Maß der Renaturierung wird die Enzymaktivität von rPA mit Hilfe des Chromozym-tPA-Assays (Roche Diagnostics) ermittelt und anhand einer Eichgerade für das native rPA quantifiziert.

In Figur 4 ist die Renaturierungsausbeute von rPA in Abhängigkeit von der Konzentration des Salzes 4-Methyl-N-butyl-pyridinium-tetrafluorborat dargestellt. Mit steigender Konzentration an 4-Methyl-N-butyl-pyridinium-tetrafluorborat steigt die Renaturierungsausbeute von rPA. Bei einer Konzentration von 94 % (v/v) 4-Methyl-N-butyl-pyridinium-tetrafluorborat liegt die Ausbeute an renaturiertem rPA bei 13,5 %.

### Beispiel 6: Renaturierung von rPA mittels 1-Butyl-3-methyl-imidazolium-tetrafluorborat

Die Renaturierung des in 6 M Guanidin, pH 2 befindlichen rPA erfolgt durch eine 1: 100 Verdünnung in 0,1 M Tris, pH 10,5, 1 mM EDTA, 5 mM GSSG, 2 mM GSH in Gegenwart unterschiedlicher Konzentrationen von 1-Butyl-3-methyl-imidazolium-tetrafluorborat. Die Renaturierung wird für mindestens 16 h bei 10°C durchgeführt. Nach der Renaturierung werden die Proben gegen das 1000fache Volumen von 0, 1 M Tris, pH 8, 1 mM EDTA über Nacht dialysiert. Als Maß der Renaturierung wird die Enzymaktivität von rPA mit Hilfe des Chromozym-tPA-Assays (Roche Diagnostics) ermittelt und anhand einer Eichgerade für das native rPA quantifiziert.

In Figur 5 ist die Renaturierungsausbeute von rPA in Abhängigkeit von der Konzentration des Salzes 1-Butyl-3-methyl-imidazolium-tetrafluorborat dargestellt. Die Renaturierungsausbeute liegt bei 8 % in Gegenwart von 20 % (v/v) 1-Butyl-3-methyl-imidazolium-tetrafluorborat.

### Beispiel 7: Aggregation von rPA während der Renaturierung

Die Renaturierung des in 6 M Guanidin, pH 2 befindlichen rPA erfolgt durch eine 1:100 Verdünnung in 0,1 M Tris, pH 10,5, 1 mM EDTA, 5 mM GSSG, 2 mM GSH in An- oder Abwesenheit von 5 % 1-Ethyl-3-methyl-imidazolium-tetxafluorborat. Die Reaktion wird bei 10°C in einer rührbaren Fluoreszenzküvette durchgeführt. Während der Kinetik wird die Aggregation des Proteins durch Messung der Lichtstreuung bei 360 nm Anregung und 360 nm Emission aufgezeichnet.

In Figur 6 ist der pufferkorrigierte Plateauwert der Aggregationsmessungen von der rPA-Renaturierung in Abwesenheit des Salzes (offener Balken) und in Gegenwart von 5 % Vol.-% 1-Ethyl-3-methyl-imidazolium-tetrafluorborat (gefüllter Balken) dargestellt. In Gegenwart von mindestens 5 Vol.-% 1-Ethyl-3-methyl-imidazolium-tetrafluorborat wird die Aggregation des zu renaturierenden rPA's nahezu vollständig unterdrückt.

### Beispiel 8: Löslichkeit der N-terminalen Domäne des GLP1-Rezeptors (nGLP1R)

Die nativ strukturierte Rezeptordomäne wurde in 0,4 M Kaliumphosphat, pH 6,3, 0,4 M Ammoniumsulfat, 20°C in An- und Abwesenheit von 5 % 1-Ethyl-3-Methyl-imidazolium chlorid bei verschiedenen Proteinkonzentrationen über Nacht inkubiert. Anschließend wurden die Proteinaggregate bei 70 000 rpm abzentrifugiert und der Anteil des löslichen Proteins mittels Absorptionsspektroskopie bei 280 nm quantifiziert. In Figur 7 sind die bei den jeweiligen Bedingungen maximal erreichbaren Konzentrationen an löslichem Protein gegenübergestellt.

Das Beispiel zeigt damit die Erhöhung der Löslichkeit bzw. der Verminderung der Aggregation durch substituierte Imidazoliumsalze.

### Beispiel 9: Thermische Stabilität von rPA

Natives rPA in einer Konzentration von 20 µg/ml in 0,1 M Tris, pH 10,5, 1 mM EDTA, 5 mM GSSG, 2 mM GSH in Gegenwart verschiedener Konzentrationen von 1-Ethyl-3-methyl-imidazolium-tetrafluorborat wird mit einer Heizrate von ca. 0,3°C/min von 20°C bis 90°C aufgeheizt. Während dieses Vorganges wird der Aggregationszustand des Proteins mittels Lichtstreuung analysiert. Hierzu wird die Probe in einem Fluorimeter bei 360 nm angeregt und das Signal ebenfalls bei 360 nm detektiert.

In Figur 8 ist das Aggregationsverhalten von rPA während thermischer Denaturierung dargestellt (gefüllte Kreise Vol.-0 %, gefüllte Dreicke 5 Vol.-%, offene Kreise 12 Vol.-%, offene Dreiecke 20 Vol.-% 1-Ethyl-3-methyl-imidazolium-tetrafluorborat). Die Aggregation von rPA in Abwesenheit von 1-Ethyl-3-methyl-imidazolium-tetrafluorborat setzt bei 55°C ein. In Gegenwart von 5 Vol.-% 1-Ethyl-3-methyl-imidazolium-tetrafluorborat ist eine verstärkte Aggregation erst oberhalb von 80°C zu beobachten, bei höheren 1-Ethyl-3-methyl-imidazolium-tetrafluorborat-Konzentrationen tritt bis 90°C keine messbare Aggregation auf.

### Beispiel 10: Renaturierung von rPA mit 3-(1-Pyridinio)-1-propansulfat (Vergleichsbeispiel)

Die Renaturierung des in 6 M Guanidin, pH 2 befindlichen rPA erfolgt durch eine 1:100 Verdünnung in 0,1 M Tris, pH 10,5, 1 mM EDTA, 5 mM GSSG, 2 mM GSH in Gegenwart unterschiedlicher Konzentrationen von 3- (1-Pyridinio)-1-propansulfat. Die Renaturierung wird für mindestens 16 h bei 10°C durchgeführt Nach der Renaturierung werden die Proben gegen das 1000 fache Volumen von 0,1 M Tris, pH 8, 1 mM EDTA über Nacht dialysiert. Als Maß der Renaturierung wird die Enzymaktivität von rPA mit Hilfe des Chromozym-tPA-Assays (Roche Diagnostics) ermittelt und anhand einer Eichgerade für das native rPA quantifiziert.

In Figur 9 ist die Renaturierungsausbeute von rPA in Abhängigkeit von der Konzentration des Salzes 3-(1-Pyridinio)-1-propansulfat dargestellt. Die maximale Renaturierungsausbeute liegt bei 18 % in Gegenwart von 30 Vol.-% 3-(1-Pyridinio)-1-propansulfat.

### Beispiel 11: Renaturierung von rPA mit Trigonellin-Hydrochlorid (Vergleichsbeispiel)

Die Renaturierung des in 6 M Guanidin, pH 2 befindlichen rPA erfolgt durch eine 1:100 Verdünnung in 0,1 M Tris, pH 10,5, 1 mM EDTA, 5 mM GSSG, 2 mM GSH in Gegenwart unterschiedlicher Konzentrationen von Trigonellin-Hydrochlorid. Die Renaturierung wird für mindestens 16 h bei 10°C durchgeführt. Nach der Renaturierung werden die Proben gegen das 1000fache Volumen von 0,1 M Tris, pH 8, 1 mM EDTA über Nacht dialysiert. Als Maß der Renaturierung wird die Enzymaktivität von rPA mit Hilfe des Chromozym-tPA-Assays (Roche Diagnostics) ermittelt und anhand einer Eichgerade für das native rPA quantifiziert.

In Figur 10 ist die Renaturierungsausbeute von rPA in Abhängigkeit von der Konzentration des Salzes Trigonellin-Hydrochlorid dargestellt. Es ist keine verbesserte Renaturierung von rPA in Gegenwart des Salzes zu beobachten.

## Patentansprüche

1. Verwendung von substituierten Imidazoliumsalzen zur Renaturierung, zur Erhöhung der thermischen Stabilität und/oder zur Verminderung der Aggregation von Proteinen.

2. Verwendung nach Anspruch 1,
**dadurch gekennzeichnet,**
**dass** die Imidazoliumsalze durch Alkyl-, Alkenyl,- Aryl-und/oder Aralkyl-Reste substituiert sind, welche wiederum durch funktionelle Gruppen substituiert sein können, bevorzugt durch Stickstoff-, Schwefel-, und/oder Phosphor-haltige Gruppen, und/oder dass die Imidazoliumsalze an beiden Stickstoffatomen des Imidazoliumrings substituiert sind, und/oder dass die Imidazoliumsalze an einem oder mehreren der Kohlenstoffatome des Imidazoliumrings substituiert sind, und/oder dass der Imidazoliumring als Substituenten C₁- C₄-Alkylreste, bevorzugt Methyl-, und/oder Ethylreste, C₁- C₄-Alkenylreste und/oder mono- oder bicyclische Arylreste aufweist, und/oder dass der Imidazoliumring an mindestens einem der N-Atome einen Methylrest aufweist, und/oder dass der Imidazoliumring einen oder mehrere der nachfolgenden Substituenten aufweist: Phenol, Biphenyl, Biphenol, Naphthalin, Naphthalincarbonsäuren, Naphthalinsulfonsäuren, Biphenylole, Biphenylcarbonsäuren, Phenol, Phenylsulfonat, Phenolsulfonsäuren und/oder substituiertes oder unsubstituiertes Benzyl, und/oder dass die Substituenten weiterhin durch ein oder mehrere der nachfolgenden Reste substituiert sind: Amine, Carboxyl-, Carbonyl-, Aldehyd-, Hydroxy-, Sulfat-, Sulfonat- und/oder Phosphat-Reste.

3. Verwendung nach Anspruch 1 oder 2,
**dadurch gekennzeichnet,**
**dass** es sich bei den Imidazoliumsalzen um bei Raumtemperatur ionische Flüssigkeiten handelt.

4. Verwendung nach einem oder mehreren der vorhergehenden Ansprüche, zur Renaturierung, Erhöhung der thermischen Stabilität und/oder Verminderung der Aggregation von:
Proteasen, bevorzugt Serin-Proteasen, insbesondere Thrombin, Faktor Xa, Caspasen, Cathepsine, Trypsin und/oder Chymotrypsin, Cystein-Proteasen, sauren Proteasen, wie Pepsin oder Rennin, Metallo-Proteasen, wie Thermolysin;
Protease-Inhibitoren, bevorzugt Pepstatine, Antipain, Chemostatine, Elastinal, Leupeptine, Bestatin, Antithrombin III ;
DNA-bindenden Proteinen, bevorzugt Transkriptionsfaktoren; insbesondere NF Kappa B und Mitglieder der Familien Jun, Fos, Krox, Myc, E2F und/oder virale T-Antigene; virale Proteine, wie virale Hüllproteine, Kapsidproteine, virale Proteasen, Polymerasen und/oder T-Antigene;
Phosphatasen;
Proteinkinasen, bevorzugt Tyrosinkinasen und/oder Serinkinasen;
Proteinen der Immunglobulin-Superfamilie, z.B. Antikörper und Fragmente hiervon; Wachstumsfaktoren, bevorzugt epidermaler Wachstumsfaktor (EGF), Erythropoeietin, Fibroblasten-Wachstumsfaktor (FGF), Insulin-ähnliche Wachstumsfaktoren I und II (IGF I und IGF II), Interleukin-2 (IL-2), Nervenwachstumsfaktor (NGF), transformierender Wachstumsfaktor-β (TGF- β) und/oder Thrombocyten-Wachstumsfaktor (PDGF);
Lysozym, rPA, alpha-Glucosidase
sowie von diesen Proteinen abgeleitete Proteine und Fragmente

5. Verfahren zur Renaturierung, Erhöhung der thermischen Stabilität und/oder Verminderung der Aggregation von Proteinen,
**dadurch gekennzeichnet,**
**dass** die zu behandelnden Proteine mit einem flüssigen Medium, welches substituierte Imidazoliumsalze enthält, in Kontakt gebracht werden.

6. Verfahren nach Anspruch 5 zur Renaturierung von Proteinen, wobei als flüssiges Medium ein flüssiges Renaturierungsmedium eingesetzt wird, und/oder dass als flüssiges Medium ein für das zu behandelnde Protein geeignetes Puffersystem eingesetzt wird, und/oder dass als Puffersubstanzen Tris, HEPES, Mes, Mops, Acetat, Glycin und/oder Phosphat eingesetzt werden, und/oder dass die Pufferkonzentration in dem flüssigen Medium 10 bis 1000 mM beträgt, bevorzugt, 5 -200 mM, noch bevorzugter 10 - 50 mM, und/oder dass der pH-Wert des Puffersystems 4-11, bevorzugt 7,5 - 10,5 beträgt.

7. Verfahren nach einem der Ansprüche 5 und 6,
**dadurch gekennzeichnet,**
**dass** die Imidazoliumsalze Alkyl-, Alkenyl,- Aryl-und/oder Aralkyl-Reste substituiert sind, welche wiederum durch funktionelle Gruppen substituiert sein können, bevorzugt durch Stickstoff-, Schwefel-, und/oder Phosphor-haltige Gruppen, und/oder dass die Imidazoliumsalze an beiden Stickstoffatomen des Imidazoliumrings substituiert sind, und/oder dass die Imidazoliumsalze an einem oder mehreren der Kohlenstoffatome des Imidazoliumrings substituiert sind, und/oder dass der Imidazoliumring als Substituenten C₁- C₄-Alkylreste, bevorzugt Methyl-, und/oder Ethylreste, C₁- C₄-Alkenylreste und/oder mono- oder bicyclische Arylreste aufweist, und/oder dass der Imidazoliumring an mindestens einem der N-Atome einen Methylrest aufweist, und/oder dass der Imidazoliumring einen oder mehrere der nachfolgenden Substituenten aufweist: Phenol, Biphenyl, Biphenol, Naphthalin, Naphthalincarbonsäuren, Naphthalinsulfonsäuren, Biphenylole, Biphenylcarbonsäuren, Phenol, Phenylsulfonat, Phenolsulfonsäuren und/oder substituiertes oder unsubstituiertes Benzyl, und/oder dass die Substituenten weiterhin durch ein oder mehrere der nachfolgenden Reste substituiert sind: Amine, Carboxyl-, Carbonyl-, Aldehyd-, Hydroxy-, Sulfat-, Sulfonat- und/oder Phosphat-Reste.

8. Verfahren nach einem oder mehreren der Ansprüche 5-7,
**dadurch gekennzeichnet,**
**dass** es sich bei den Imidazoliumsalzen um bei Raumtemperatur ionische Flüssigkeiten handelt.

9. Verfahren nach einem oder mehreren der Ansprüche 5-7,
**dadurch gekennzeichnet,**
**dass** das Inkontaktbringen erfolgt, indem das zu behandelnde Protein mit dem flüssigen Medium verdünnt, dialysiert und/oder diafiltriert wird.

10. Verfahren nach einem oder mehreren der Ansprüche 5-9,
**dadurch gekennzeichnet,**
**dass** die Proteinkonzentration des zu behandelnden Proteins beim Inkontaktbringen 5 - 500 µg/ml, bevorzugt 10 - 200 µg/ml, noch bevorzugter ungefähr 50-100 µg/ml, beträgt, und/oder dass die Konzentration an substituierten Imidazoliumsalzen, bezogen auf das flüssige Medium, 5 - 95 Vol.-% beträgt, bevorzugt 5 - 50 Vol.%, am bevorzugtesten 10 - 40 Vol.%, und/oder daß die Molarität an substituierten Imidazoliumsalzen, bezogen auf das flüssige Medium, 0,25 - 5 M beträgt, bevorzugt 0,25 - 3,5 M, am bevorzugtesten 0,5 - 3 M.

11. Verfahren nach einem oder mehreren der Ansprüche 5-10,
**dadurch gekennzeichnet,**
**dass** das Verfahren durchgeführt wird, indem das zu renaturierende Protein dem Renaturierungsmedium mehrfach nacheinander oder kontinuierlich zugeführt wird, und/oder dass das Inkontaktbringen der zu renaturierenden Proteine mit dem Renaturierungsmedium für 0,1 - 100 h, bevorzugt, 1 - 50 h, noch bevorzugter 5 - 20 h erfolgt.

12. Verfahren nach einem oder mehreren der Ansprüche 5-11, zur Renaturierung, Erhöhung der thermischen Stabilität und/oder Verminderung der Aggregation von:
Proteasen, bevorzugt Serin-Proteasen, insbesondere Thrombin, Faktor Xa, Caspasen, Cathepsine, Trypsin und/oder Chymotrypsin, Cystein-Proteasen, sauren Proteasen, wie Pepsin oder Rennin, Metallo-Proteasen, wie Thermolysin;
Protease-Inhibitoren, bevorzugt Pepstatine, Antipain, Chemostatine, Elastinal, Leupeptine, Bestatin, Antithrombin III;
DNA-bindenden Proteinen, bevorzugt Transkriptionsfaktoren; insbesondere NF Kappa B und Mitglieder der Familien Jun, Fos, Krox, Myc, E2F, virale T-Antigene;
virale Proteine, wie virale Hüllproteine, Kapsidproteine, virale Proteasen, Polymerasen und/oder T-Antigene;
Phosphatasen;
Proteinkinasen, bevorzugt Tyrosinkinasen und/oder Serinkinasen;
Proteinen der Immunglobulin-Superfamilie, z.B. Antikörper und Fragmente hiervon;
Wachstumsfaktoren, bevorzugt epidermaler Wachstumsfaktor (EGF), Erythropoeietin, Fibroblasten-Wachstumsfaktor (FGF), Insulin-ähnliche Wachstumsfaktoren I und II (IGF I und IGF II), Interleukin-2 (IL-2), Nervenwachstumsfaktor (NGF), transformierender Wachstumsfaktor-β (TGF- β) und Thrombocyten-Wachstumsfaktor (PDGF);
Lysozym, rPA, alpha-Glucosidase
sowie von diesen Proteinen abgeleitete Proteine und Fragmente.

13. Verfahren nach einem oder mehreren der Ansprüche 5-12,
**dadurch gekennzeichnet,**
**dass** disulfidfreie Proteine renaturiert werden,
wobei das Inkontaktbringen des disulfidfreien Proteins mit dem Renaturierungsmedium in Gegenwart eines Reduktionsmittels erfolgt, vorzugsweise DTT, DTE und/oder Cystein, und/oder dass disulfidverbrückte Proteine renaturiert werden,
wobei das Inkontaktbringen des disulfidverbrückten Proteins mit dem Renaturierungsmedium in Gegenwart eines Redoxsystems aus oxidierten und reduzierten Thiolsubstanzen wie DTT, DTE, Glutathion, Cystein und/oder Mercaptoethanol erfolgt, und/oder dass das Konzentrationsverhältnis von reduzierten Substanzen zu oxidierten Substanzen 1:10 bis 20:1, bevorzugt 1:5 bis 10 : 1, weiterhin bevorzugt 1:1 bis 5:1, beträgt, und/oder dass das flüssige Medium zur Renaturierung von Proteinen weitere Renaturierungssubstanzen enthält, bevorzugt Harnstoff, Guanidin, L-Arginin, Alkylharnstoff, Carbonsäureamide, alkylierte Amine, Tris-Puffer, Polyethylenglykol und/oder Detergenzien.

## Claims

1. Use of substituted imidazolium salts for the renaturation, for the increase of the thermal stability and/or for the decrease of aggregation of proteins.

2. The use according to claim 1,
**characterized in that**
said imidazolium salts are substituted by alkyl, alkenyl, aryl and/or aralkyl groups which may themselves be substituted by functional groups, preferably by groups containing nitrogen, sulfur and/or phosphor, and/or
said imidazolium salts are substituted at both nitrogen atoms of the imidazolium ring, and/or
said imidazolium salts are substituted at one or more of the carbon atoms of the imidazolium ring, and/or
said imidazolium ring has C₁-C₄ alkyl groups, preferably methyl and/or ethyl groups, C₁-C₄ alkenyl groups, and/or mono- or bicyclic aryl groups as said substituents, and/or said imidazolium ring has a methyl group at at least one of the N atoms, and/or
said imidazolium ring has one or more of the following substituents: phenol, biphenyl, biphenol, naphthalene, naphthalene carboxylic acids, naphthalene sulfonic acids, biphenylols, biphenyl carboxylic acids, phenol, phenyl sulfonate, phenol sulfonic acids and/or substituted or unsubstituted benzyl, and/or
said substituents are further substituted by one or more of the following groups: amine, carboxyl, carbonyl, aldehyde, hydroxy, sulfate, sulfonate and/or phosphate groups.

3. The use according to claim 1 or 2,
**characterized in that**
said imidazolium salts are ionic liquids at room temperature.

4. The use according to one or more of the preceding claims for the renaturation, the increase of the thermal stability and/or the decrease of the aggregation of:
proteases, preferably serine proteases, particularly thrombin, factor Xa, caspases, cathepsins, trypsin and/or chymotrypsin, cysteine proteases, acidic proteases such as pepsin or rennin, metalloproteinases such as thermolysin ;
protease inhibitors, preferably pepstatins, antipain, chemostatins, elastinal, leupeptins, bestatin, antithrombin III ;
DNA binding proteins, preferably transcription factors, particularly NF kappa B and members of the jun, fos, krox, myc, E2F families, and/or viral T antigens;
viral proteins such as viral envelope proteins, capsid proteins, viral proteases, polymerases and/or T antigens;
phosphatases;
protein kinases, preferably tyrosine kinases and/or serine kinases;
proteins of the immunoglobulin superfamily, e.g. antibodies and fragments thereof; growth factors, preferably epidermal growth factor (EGF), erythropoietin, fibroblast growth factor (FGF), insulin-like growth factors I and II (IGF I and IGF II), interleukin-2 (IL-2), nerve growth factor (NGF), transforming growth factor β (TGF-β) and/or thrombocyte growth factor (PDGF);
lysozyme, rPA, alpha-glucosidase,
as well as proteins and fragments derived from these proteins.

5. A method for the renaturation, the increase of the thermal stability and/or the decrease of the aggregation of proteins,
**characterized in that**
the proteins to be treated are contacted with a liquid medium containing substituted imidazolium salts.

6. The method according to claim 5 for the renaturation of proteins wherein a liquid renaturation medium is used as the liquid medium, and/or
a buffer system suitable for the protein to be treated is used as the liquid medium, and/or
Tris, HEPES, Mes, Mops, acetate, glycine and/or phosphate are used as buffer substances, and/or
the buffer concentration in the liquid medium is 10-1000 mM, preferably 5-200 mM, still more preferred 10-50 mM, and/or
the pH value of the buffer system is 4-11, preferably 7.5-10.5.

7. The method according to one of claims 5 and 6,
**characterized in that**
said imidazolium salts are substituted by alkyl, alkenyl, aryl and/or aralkyl groups which may themselves be substituted by functional groups, preferably by groups containing nitrogen, sulfur and/or phosphor, and/or
said imidazolium salts are substituted at both nitrogen atoms of the imidazolium ring, and/or
said imidazolium salts are substituted at one or more of the carbon atoms of the imidazolium ring, and/or
said imidazolium ring has C₁-C₄ alkyl groups, preferably methyl and/or ethyl groups, C₁-C₄ alkenyl groups, and/or mono- or bicyclic aryl groups as said substituents, and/or said imidazolium ring has a methyl group at at least one of the N atoms, and/or
said imidazolium ring has one or more of the following substituents: phenol, biphenyl, biphenol, naphthalene, naphthalene carboxylic acids, naphthalene sulfonic acids, biphenylols, biphenyl carboxylic acids, phenol, phenyl sulfonate, phenol sulfonic acids and/or substituted or unsubstituted benzyl, and/or
said substituents are further substituted by one or more of the following groups: amine, carboxyl, carbonyl, aldehyde, hydroxy, sulfate, sulfonate and/or phosphate groups.

8. The method according to one or more of claims 5-7,
**characterized in that**
said imidazolium salts are ionic liquids at room temperature.

9. The method according to one or more of claims 5-7,
**characterized in that**
the contacting is performed by diluting, dialyzing and/or diafiltrating the protein to be treated with the liquid medium.

10. The method according to one or more of claims 5-9,
**characterized in that**
the protein concentration of the protein to be treated during contacting is 5-500 µg/ml, preferably 10-200 µg/ml, still more preferably about 50-100 µg/ml, and/or
the concentration of substituted imidazolium salts is 5-95 vol. %, preferably 5-50 vol. %, most preferred 10-40 vol. % based on the liquid medium, and/or
the molarity of substituted imidazolium salts is 0.25-5 M, preferably 0.25-3.5 M, most preferably 0.5-3 M based on the liquid medium.

11. The method according to one or more of claims 5-10,
**characterized in that**
the method is performed by adding the protein to be renatured to the renaturation medium at several successive times or in a continuous manner, and/or
the contacting of the proteins to be renatured with the renaturation medium is performed for 0.1-100 h, preferably 1-50 h, still more preferred 5-20 h.

12. The method according to one or more of claims 5-11 for the renaturation, the increase of the thermal stability and/or the decrease of the aggregation of:
proteases, preferably serine proteases, particularly thrombin, factor Xa, caspases, cathepsins, trypsin and/or chymotrypsin, cysteine proteases, acidic proteases such as pepsin or rennin, metalloproteases such as thermolysin;
protease inhibitors, preferably pepstatins, antipain, chemostatins, elastinal, leupeptins, bestatin, antithrombin III;
DNA binding proteins, preferably transcription factors, particularly NF kappa B and members of the jun, fos, krox, myc, E2F families, viral T antigens;
viral proteins such as viral envelope proteins, capsid proteins, viral proteases, polymerases and/or T antigens;
phosphatases;
protein kinases, preferably tyrosine kinases and/or serine kinases;
proteins of the immunoglobulin superfamily, e.g. antibodies and fragments thereof; growth factors, preferably epidermal growth factor (EGF), erythropoietin, fibroblast growth factor (FGF), insulin-like growth factors I and II (IGF I and IGF II), interleukin-2 (IL-2), nerve growth factor (NGF), transforming growth factor β (TGF-β) and thrombocyte growth factor (PDGF);
lysozyme, rPA, alpha-glucosidase,
as well as proteins and fragments derived from these proteins.

13. The method according to one or more of claims 5-12,
**characterized in that**
disulfide-free proteins are renatured wherein the contacting of the disulfide-free protein with the renaturation medium is performed in the presence of a reducing agent, preferably DTT, DTE and/or cysteine, and/or
disulfide-bridged proteins are renatured wherein the contacting of said disulfide-bridged protein with the renaturation medium is performed in the presence of a redox system composed of reduced and oxidized thiol substances such as DTT, DTE, glutathione, cysteine, and/or mercaptoethanol, and/or
the concentration ratio of reduced substances to oxidized substances is 1:10 to 20:1, preferably 1:5 to 10:1, further preferred 1:1 to 5:1, and/or
the liquid medium for the renaturation of proteins contains other renaturation substances, preferably urea, guanidinium, L-arginine, alkyl urea, carboxylic acid amides, alkylated amines, Tris buffers, polyethylene glycol and/or detergents.

## Revendications

1. Utilisation de sels d'imidazolium substitués pour la renaturation, pour l'augmentation de la stabilité thermique et/ou pour la diminution de l'agrégation des protéines.

2. Utilisation selon la revendication 1, **caractérisée en ce que** les sels d'imidazolium sont substitués par des radicaux alkyle, alcényle, aryle et/ou aralkyle qui peuvent à leur tour être substitués par des groupes fonctionnels, de préférence par des groupes azotés, soufrés et/ou phosphorés, et/ou **en ce que** les sels d'imidazolium sont substitués sur les deux atomes d'azote du cycle imidazolium, et/ou **en ce que** les sels d'imidazolium sont substitués sur un ou plusieurs des atomes de carbone du cycle imidazolium, et/ou **en ce que** le cycle imidazolium comporte comme substituants des radicaux alkyle en C₁-C₄, de préférence des radicaux méthyle et/ou éthyle, des radicaux alcényle en C₁-C₄ et/ou des radicaux aryle mono- ou bicycliques, et/ou **en ce que** le cycle imidazolium comporte un radical méthyle sur au moins l'un des atomes d'azote, et/ou **en ce que** le cycle imidazolium comporte un ou plusieurs des substituants suivants : phénol, biphényle, biphénol, naphtalène, acides naphtalènecarboxyliques, acides naphtalènesulfoniques, biphénylols, acides biphénylcarboxyliques, phénol, phénylsulfonate, acides phénylsulfoniques et/ou benzyle substitué ou non substitué, et/ou en ce que les substituants en outre sont substitués par un ou plusieurs des radicaux suivants : des radicaux amino, carboxy, carbonyle, aldéhyde, hydroxy, sulfate, sulfonate et/ou phosphate.

3. Utilisation selon la revendication 1 ou 2, **caractérisée en ce que** les sels d'imidazolium sont des liquides ioniques à la température ambiante.

4. Utilisation selon une ou plusieurs des revendications précédentes, pour la renaturation, l'augmentation de la stabilité thermique et/ou la diminution de l'agrégation de :
protéases, de préférence de sérine-protéases, en particulier la thrombine, le facteur Xa, les caspases, les cathepsines, la trypsine et/ou la chymotrypsine, de cystéine-protéases, de protéases acides telles que la pepsine ou la rennine, de métalloprotéases, telles que la thermolysine ;
d'inhibiteurs de protéases, de préférence les pepstatines, l'antipaïne, les chémostatines, l'élastinal, les leupeptines, la bestatine, l'antithrombine III ;
de protéines se liant à l'ADN, de préférence les facteurs de transcription, en particulier NF Kappa B et les membres des familles Jun, Fos, Krox, Myc, E2F et/ou les antigènes T viraux ;
de protéines virales, telles que les protéines d'enveloppe virale, les protéines nucléocapsidiques, les protéases virales, les polymérases et/ou les antigènes T ;
de phosphatases ;
de protéine kinases, de préférence les tyrosine kinases et/ou les sérine kinases ;
de protéines de la superfamille des immunoglobulines, par exemple d'anticorps et fragments de ceux-ci ;
de facteurs de croissance, de préférence le facteur de croissance de l'épiderme (EGF), l'érythropoïétine, le facteur de croissance des fibroblastes (FGF), les facteurs de croissance ressemblant à l'insuline I et II (IGF I et IGF II), l'interleukine 2 (IL-2), le facteur de croissance des neurones (NGF), le facteur transformant de croissance β (TGF-β) et/ou le facteur de croissance des thrombocytes (PDGF) ;
du lysozyme, du rPA, de l'alpha-glucosidase
ainsi que de protéines et fragments dérivés de ces protéines.

5. Procédé pour la renaturation, l'augmentation de la stabilité thermique et/ou la diminution de l'agrégation des protéines, **caractérisé en ce qu'**on met les protéines à traiter en contact avec un milieu liquide qui contient des sels d'imidazolium substitués.

6. Procédé selon la revendication 5 pour la renaturation de protéines, **caractérisé en ce qu'**on utilise comme milieu liquide un milieu liquide de renaturation, et/ou **en ce qu'**on utilise comme milieu liquide un système tampon approprié à la protéine à traiter, et/ou **en ce qu'**on utilise comme substances tampon les tampons Tris, HEPES, Mes, Mops, acétate, glycine et/ou phosphate, et/ou **en ce que** la concentration de tampon dans le milieu liquide va de 10 à 1 000mM, de préférence de 5 à 200 mM, encore mieux de 10 à 50 mM, et/ou **en ce que** le pH du système tampon est de 4-11, de préférence de 7,5-10,5.

7. Procédé selon la revendication 5 ou 6, **caractérisé en ce que** les sels d'imidazolium sont substitués par des radicaux alkyle, alcényle, aryle et/ou aralkyle qui peuvent à leur tour être substitués par des groupes fonctionnels, de préférence par des groupes azotés, soufrés et/ou phosphorés, et/ou **en ce que** les sels d'imidazolium sont substitués sur les deux atomes d'azote du cycle imidazolium, et/ou **en ce que** les sels d'imidazolium sont substitués sur un ou plusieurs des atomes de carbone du cycle imidazolium, et/ou **en ce que** le cycle imidazolium comporte comme substituants des radicaux alkyle en C₁-C₄, de préférence des radicaux méthyle et/ou éthyle, des radicaux alcényle en C₁-C₄ et/ou des radicaux aryle mono- ou bicycliques, et/ou **en ce que** le cycle imidazolium comporte un radical méthyle sur au moins l'un des atomes d'azote, et/ou **en ce que** le cycle imidazolium comporte un ou plusieurs des substituants suivants : phénol, biphényle, biphénol, naphtalène, acides naphtalènecarboxyliques, acides naphtalènesulfoniques, biphénylols, acides biphénylcarboxyliques, phénol, phénylsulfonate, acides phénylsulfoniques et/ou benzyle substitué ou non substitué, et/ou en ce que les substituants en outre sont substitués par un ou plusieurs des radicaux suivants: des radicaux amino, carboxy, carbonyle, aldéhyde, hydroxy, sulfate, sulfonate et/ou phosphate.

8. Procédé selon une ou plusieurs des revendications 5 à 7, **caractérisé en ce que** les sels d'imidazolium sont des liquides ioniques à la température ambiante.

9. Procédé selon une ou plusieurs des revendications 5 à 7, **caractérisé en ce que** la mise en contact s'effectue par dilution, dialyse et/ou diafiltration de la protéine à traiter avec le milieu liquide.

10. Procédé selon une ou plusieurs des revendications 5 à 9, **caractérisé en ce que** lors de la mise en contact la concentration de la protéine à traiter est de 5-500 µg/ml, de préférence de 10-200 µg/ml, encore mieux d'environ 50-100 µg/ml, et/ou **en ce que** la concentration des sels d'imidazolium substitués, par rapport au milieu liquide, est de 5-95 % en volume, de préférence de 5-50 % en volume, encore mieux de 10-40 % en volume, et/ou **en ce que** la molarité des sels d'imidazolium substitués, par rapport au milieu liquide, est de 0,25-5 M, de préférence de 0,25-3,5 M, encore mieux de 0,5-3 M.

11. Procédé selon une ou plusieurs des revendications 5 à 10, **caractérisé en ce qu'**on effectue le procédé en ajoutant plusieurs fois successivement ou en continu la protéine à renaturer au milieu de renaturation, et/ou **en ce qu'**on effectue la mise en contact de la protéine à renaturer avec le milieu de renaturation pendant 0,1-100 heures, de préférence 1-50 heures, encore mieux 5-20 heures.

12. Procédé selon une ou plusieurs des revendications 5 à 11, pour la renaturation, l'augmentation de la stabilité thermique et/ou la diminution de l'agrégation de:
protéases, de préférence de sérine-protéases, en particulier la thrombine, le facteur Xa, les caspases, les cathepsines, la trypsine et/ou la chymotrypsine, de cystéine-protéases, de protéases acides telles que la pepsine ou la rennine, de métalloprotéases, telles que la thermolysine ;
d'inhibiteurs de protéases, de préférence les pepstatines, l'antipaïne, les chémostatines, l'élastinal, les leupeptines, la bestatine, l'antithrombine III ;
de protéines se liant à l'ADN, de préférence les facteurs de transcription, en particulier NF Kappa B et les membres des familles Jun, Fos, Krox, Myc, E2F et/ou les antigènes T viraux ;
de protéines virales, telles que les protéines d'enveloppe virale, les protéines nucléocapsidiques, les protéases virales, les polymérases et/ou les antigènes T ;
de phosphatases ;
de protéine kinases, de préférence les tyrosine kinases et/ou les sérine kinases ;
de protéines de la superfamille des immunoglobulines, par exemple d'anticorps et fragments de ceux-ci ;
de facteurs de croissance, de préférence le facteur de croissance de l'épiderme (EGF), l'érythropoïétine, le facteur de croissance des fibroblastes (FGF), les facteurs de croissance ressemblant à l'insuline I et II (IGF 1 et IGF II), l'interleukine 2 (IL-2), le facteur de croissance des neurones (NGF), le facteur transformant de croissance β (TGF-β) et/ou le facteur de croissance des thrombocytes (PDGF) ;
du lysozyme, du rPA, de l'alpha-glucosidase
ainsi que de protéines et fragments dérivés de ces protéines.

13. Procédé selon une ou plusieurs des revendications 5 à 12, **caractérisé en ce que** des protéines exemptes de ponts disulfure sont renaturées, la mise en contact de la protéine exempte de ponts disulfures avec le milieu de renaturation s'effectuant en présence d'un réducteur, de préférence le DTT (dithiothréitol), le DTE (dithioérythritol) et/ou la cystéine, et/ou **en ce que** des protéines à ponts disulfure sont renaturées, la mise en contact de la protéine à ponts disulfure avec le milieu de renaturation s'effectuant en présence d'un système redox constitué de substances de type thiol oxydées et réduites, telles que le DTT, le DTE, le glutathion, la cystéine et/ou le mercaptoéthanol, et/ou **en ce que** le rapport de concentrations des substances réduites aux substances oxydées va de 1:10 à 20:1, de préférence de 1:5 à 10:1 de façon particulièrement préférée de 1:1 à 5:1, et/ou **en ce que** le milieu liquide pour la renaturation de protéines contient d'autres substances de renaturation, de préférence de l'urée, de la guanidine, de la L-arginine, une alkylurée, des carboxamides, des amines alkylées, du tampon Tris, du polyéthylèneglycol et/ou des détergents.
